# EUROPÄISCHE PATENTANMELDUNG

(11) **EP 1 921 094 A2**
(43) Veröffentlichungstag der Anmeldung: **14.05.2008**
(21) Anmeldenummer: 07122292.1
(22) Anmeldetag: 14.04.1998
(51) Int. Cl.: C08F 8/00, C08G 81/02, G01N 33/545, A61K 31/74

(54) **Interaktionssystem zur Präsentation und Entfernung von Substanzen**

(30) Priorität: 14.04.1997 DE 19715504
(62) Teilanmeldung aus: 98922710.3
(71) Anmelder: Max-Planck-Gesellschaft zur Förderung der Wissenschaften e.V. Berlin, 80539 München (DE)
(72) Erfinder: Nowak, Götz, Prof. Dr., 99097 Erfurt (DE); Bucha, Elke, Dr., 99094 Erfurt (DE)
(74) Vertreter: Vossius & Partner

(57) **Zusammenfassung**

Die Erfindung betrifft die Verwendung einer Oberfläche aus einem Kunststoff auf der Basis von Monomeren, die mindestens ein von einer Carbonsäure abgeleitetes Strukturelement (A) enthalten, wobei der Kunststoff ein Poly(meth)acrylat, ein Polyvinylester oder ein Mischpolymer oder Gemisch davon mit anderen polymersierbaren Substanzen ist zur Entfernung eines Linkers mit einer daran gekoppelten physiologisch und/oder pharmakologisch aktiven Substanz aus einer Flüssigkeit, wobei der Linker ausgewählt ist aus (Poly)alkylenglykolen, (Poly)alkyleniminen, (Poly)alkylenaminen, (Poly)alkylensulfiden und Polyoxazolinen und mindestens ein Strukturelement (B), das zur Bildung von Wasserstoffbrücken fähig ist, aufweist, wobei über Wasserstoffbrückenbindungen eine stabile Wechselwirkung zwischen dem Strukturelement (A) der Oberfläche und dem Strukturelement (B) des Linkers entsteht, die durch pH-Werte im Bereich von 2 bis 13 oder Temperaturen bis 60°C nicht aufgehoben werden kann.

## Beschreibung

Die vorliegende Erfindung betrifft Interaktionssysteme zur Präsentation und Entfernung von Substanzen in Flüssigkeiten. Die Erfindung betrifft ferner mit diesen Interaktionssystemen hergestellte Vorrichtungen wie beispielsweise Kapillarschlauchsysteme, Fasermaterial zur Filterung von physiologischen Flüssigkeiten, Filtermatten, Gelenk- oder Gefäßprothesen, etc.

Zu den verschiedensten Zwecken ist es häufig erforderlich, aus einer Flüssigkeit gezielt Substanzen zu entfernen bzw. Substanzen in einer Flüssigkeit zu präsentieren.

Beispielsweise werden häufig zur Erhöhung des Molekulargewichts und damit zur Verbesserung der Pharmakokinetik im Körper physiologische Wirksubstanzen mit Polyalkylenglykolen, z.B. Polyethylenglykol, gekoppelt (vgl. z.B. Thrombosis and Haemostasis, 77 (1), 168-73 (1997), Peptide Research, Vol. 8, No. 2 (1995)). Derartige Substanzen finden mittlerweile eine breite therapeutische Anwendung. Bisher stehen für derartige Substanzen keine wirksamen funktionellen Antidote bzw. die Wirkung neutralisierende Systeme, d.h. Systeme zur Entfernung dieser Substanzen, zur Verfügung.

Zelluläre Signal-Substanzen einer gestörten Funktion werden bei schweren Erkrankungen sehr häufig in das Blut abgegeben, wodurch sich diese zellulären Signalfaktoren an jede Stelle des Körpers rasch verteilen können. Dadurch können sowohl sinnvolle, häufig aber auch pathologische Antworten des Organismus auf derartige Signale entstehen. Durch Neutralisierung oder Blockierung dieser pathogenetischen Faktoren kann eine Krankheit unterbrochen werden, bzw. ein Fortschreiten aufgehalten werden, so daß körpereigenen Repairmechanismen die Gelegenheit gegeben wird, ursächlich einzugreifen.

Typische Beispiele für solche Signalstoffe sind die zellulären Boten der endothelialen und zirkulierenden Zellen des Blutes wie z.B. CD1, CD2, CD6, CD8, CD16, Tumornekrosefaktor (TNF) etc. Auch pathogenetisch bedeutsame, induzierte Proteine wie z.B. das Lipoprotein-bindende Protein LBP sind für die Entwicklung von schwersten Komplikationen bei septischen Schockpatienten verantwortlich.

Es wäre wichtig, derartige Substanzen über ein extrakorporales therapeutisches System schonend entfernen zu können, ohne den Organismus mit weiteren Substanzen zu belasten. Weiterhin wäre die Therapie von bestimmten Erkrankungen mit Systemen zur Präsentation von monoklonalen Antikörpern bzw. Fragmenten dieser monoklonalen Antikörper vorteilhaft. Augenblicklich ist eine derartige Therapie nur kurzfristig anwendbar, weil die immunkompetenten Zellen des Organismus sehr rasch humane Antikörper gegen diese Fremdproteine produzieren. Eine lokale Präsentation derartiger monoklonaler Antikörper in der Blutzirkulation kann ohne Immunreaktivität lange Zeit zur Neutralisierung von Antigenen im Blut verwendet werden.

Ferner ist es oft wünschenswert, bestimmte Lebensmittelinhaltsstoffe, wie beispielsweise Cholesterin, bestimmte Fettsäuren, Alkohol, Verunreinigungen mikrobieller bzw. bakterieller Art etc. schonend und einfach entfernen zu können, z.B. um gezielt Lebensmittel für spezielle Diätanforderungen herstellen zu können.

Der vorliegenden Erfindung liegt daher die Aufgabe zugrunde, ein einfaches kostengünstiges und universell anwendbares System zur Entfernung bzw. Präsentation von Substanzen in Flüssigkeit bereitzustellen. Das System soll den jeweiligen Erfordernissen der Praxis leicht angepaßt werden können. Ferner soll das System die zu behandelnde Flüssigkeit bzw. Umgebung nicht nachteilig verändern und aus dieser einfach zu entfernen sein. Sofern das System für diagnostische und therapeutische Zwecke verwendet wird, soll es schonend anwendbar sein und den Körper nicht belasten.

Gelöst wird die Aufgabe erfindungsgemäß durch ein Interaktionssystem, umfassend mindestens eine aktive Oberfläche aus einem Kunststoff auf der Basis von Monomeren, die mindestens ein von einer Carbonsäure abgeleitetes Strukturelement (A) enthalten, und mindestens eine an einen Linker mit mindestens einem zur Wasserstoffbrückenbindung fähigen Strukturelement (B) gekoppelte Substanz, wobei die Interaktion zwischen dem Strukturelement (A) und (B) stattfindet. Bevorzugt befindet sich das von einer Carbonsäure abgeleitete Strukturelement in der Seitenkette des Monomeren. Besonders bevorzugt umfaßt der Kunststoff das Strukturelement 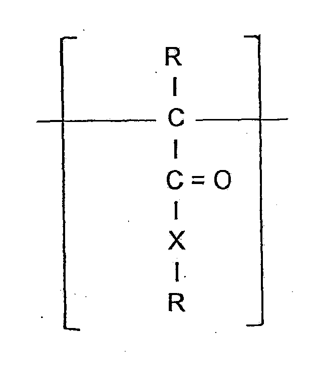 worin die Reste R gleich oder verschieden sein können und für einen beliebigen Alkyl- oder Arylrest oder ein Wasserstoffatom stehen. Der Rest X ist fakultativ und bedeutet O, N oder CH₂.

Überraschenderweise wurde bei Untersuchungen zur Bindungsfähigkeit von Oberflächenstrukturen gefunden, daß Oberflächen aus einem Kunststoff auf der Basis von Monomeren, die mindestens ein von einer Carbonsäure abgeleitetes Strukturelement (A) enthalten, eine sehr stabile Wechselwirkung mit Linkern mit mindestens einem zur Wasserstoffbrückenbindung fähigen Strukturelement eingehen. Eine derartige Wechselwirkung ist außerordentlich stabil und kann beispielsweise durch pH-Werte von 2 bis 13 bzw. Puffer mit hoher Ionenstärke oder Temperaturen bis +60°C nicht aufgehoben werden. Diese Wechselwirkung läßt sich nun zur Ankopplung der verschiedensten Substanzen nutzen, indem die betreffenden Substanzen an diesen Linker gekoppelt werden und dann mit dem Kunststoff eine entsprechende Wechselwirkung eingehen. Aus derartigen Kunststoffen lassen sich aktive Oberflächen herstellen, d.h. Oberflächen, an denen eine entsprechende Wechselwirkung und anschließende Reaktion der angelagerten Substanz mit einem Reaktionspartner stattfindet. Diese Oberfläche kann in beliebiger Form und Dimensionierung vorliegen. Sie ist bevorzugt eine Membran, ein poröses oder solides Mikropartikel, ein magnetisches Mikropartikel, ein versponnenes Material oder eine Beschichtung aus einer natürlichen oder synthetischen Substanz. Es sind auch Kombinationen möglich. So kann man z.B. Mikropartikel in Fasermaterial einweben bzw. damit assoziieren. So entsteht eine Netz-/Gerüststruktur, wodurch ein Absacken oder Zusammenpressen der Mikropartikel z.B. in Chromatographiesäulen vermieden wird. Diese aktive Oberfläche kann in verschiedenen Formen beispielsweise in Systeme wie Kapillarschlauchsysteme, Filter für physiologische Flüssigkeiten, Hämodialysatoren, physiologische Ersatzflüssigkeiten, Enzymdeliverysysteme, Gelenk- oder Gefäßprothesen oder künstliche Organe eingearbeitet werden. So können beispielsweise poröse Mikropartikel als aktive Oberfläche auf herkömmlichen Schlauch- und Filtersystemen aufgebracht werden. Es lassen sich so beliebige, universell anwendbare Systeme zusammenstellen. Durch die Möglichkeit des einfachen Primens können oft schwierig durch eine kovalente Bindung zu koppelnde Substanzen leicht auf den entsprechenden Oberflächen aufgebracht werden. Dies ist besonders vorteilhaft, wenn entsprechende apparative Ausrüstungsgegenstände sterilisiert werden sollen. Es ist oft schierig, kovalent gebundene Substanzen ohne Schädigung zu sterilisieren. Erfindungsgemäß lassen sich Kunststoffoberfläche und parallel dazu die an einen Linker gekoppelte Verbindung sterilisieren und dann können beide sterilen Komponenten wieder unter sterilen Bedingungen einfach miteinander zur Wechselwirkung gebracht werden.

Zusammen mit der an einen Linker mit mindestens einem zur Wasserstoffbrückenbindung fähigen Strukturelement gekoppelten Substanz ergibt sich ein Interaktionssystem. Das Interaktionssystem kann getrennt, d.h. in verschiedenen Phasen, oder als Reaktionseinheit vorliegen. Ferner kann die aktive Oberfläche auch zu Hohlfasern, Garnen, Preßplatten, Filtermatten, Vliesgeweben oder dergleichen versponnen werden. Wenn die aktive Oberfläche in Form von Mikropartikeln vorliegt, so können diese jede beliebige Form, z.B. kugelförmig, rautenförmig, hantelförmig, rhombenförmig etc., annehmen. Sie sind jedoch bevorzugt sphärisch mit einem Durchmesser von 0,5 bis 500 *µ*m, bevorzugt 1 bis 300 *µ*m, weiter bevorzugt 1 bis 250 *µ*m. Bevorzugt sind die Mikropartikel porös und besitzen somit eine größere Oberfläche.

Erfindungsgemäß umfaßt der Kunststoff mindestens das Strukturelement (A)

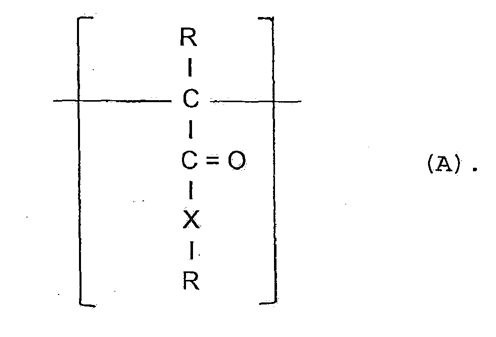

Dieses Strukturelement ist beispielsweise Teil eines Polymeren der allgemeinen Formel (I) 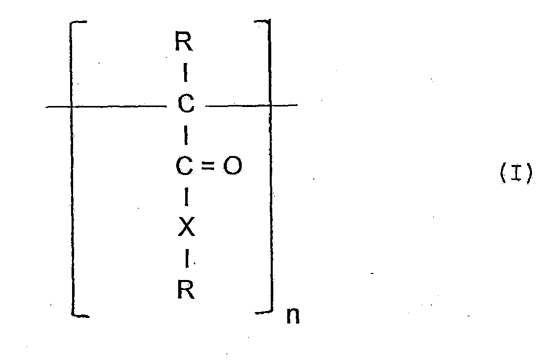 worin R einen Alkyl- oder Arylrest oder ein Wasserstoffatom bedeutet, und n den Wert 10 bis 10.000 besitzt. Der Rest X ist fakultativ und bedeutet O, N oder CH₂.

Der Alkylrest kann jeder beliebige geradkettige oder verzweigtkettige, gegebenenfalls substituierte Alkylrest sein. Bevorzugt ist ein geradkettiger oder verzweigter, gegebenenfalls substituierter C₁₋₈ Alkylrest, wie beispielsweise eine Methyl-, Ethyl-, n-Propyl-, i-Propyl-, n-Butyl-, i-Butyl-, s-Butyl- oder t-Butyl-Gruppe. Beispiele für gegebenenfalls vorhandene Substituenten umfassen ein oder mehrere Halogenatom(e), z.B. Fluor-, Chlor-, Brom- oder Iodatome, oder Hydroxylgruppe(n), Alkylreste wie C₁₋₆-Alkylreste oder Alkoxyreste, wie z.B. C₁₋₆-Alkoxyreste, z.B. C₁₋₃-Alkoxy, wie Methoxy- oder Ethoxygruppen, oder Thiolgruppen, wie beispielsweise C₁₋₆-Alkylthiolgruppen, z.B. C₁₋₃-Alkylthiogruppen, wie Methylthio- oder Ethylthiogruppen. Der Arylrest kann ein monocyclischer oder bicyclischer, gegebenenfalls substituierter Arylrest sein, der gegebenenfalls ein oder mehrere Heteroatome enthalten kann. Beispiele hierfür sind Phenyl, 1-oder 2-Naphthyl-, Indenyl- oder Isoindenylreste. Gegebenenfalls können die Arylreste auch substituiert sein. Beispiele für heteroatomhaltige Arylreste sind ein C₁₋₉-Heteroarylrest, beispielsweise ein gegebenenfalls substituierter C₃₋₉-Heteroarylrest, der beispielsweise 1, 2, 3 oder mehrere Heteroatome, ausgewählt aus Sauerstoff, Schwefel oder Stickstoffatomen, enthält. Monocyclische Heteroarylreste umfassen beispielsweise Pyrolyl-, Furyl-, Thienyl-, Imidazolyl-, N-Methylimidazolyl-, N-Ethylimidazolyl-, Oxazolyl-, Disoxazolyl-, Thiazolyl-, Isothiazolyl-, Pyrazolyl-, 1,2,3-Triazolyl-, Benzofuryl-, Isobenzofuryl-, Benzothienyl-, Isobenzothienyl-, Indolyl-, Isoindolyl-, Benzimidazolyl-, Benzothiazolyl-, Chinazolinyl-, Naphthylpyridinyl-, Chinolinyl-, Isochinolinyl-, Tetrazolylgruppen.

Die Taktizität des so erhaltenen Polymeren kann beliebig sein, beispielsweise isotaktisch, heterotaktisch bzw. syndiotaktisch.

Der Linker enthält mindestens ein zur Wasserstoffbrückenbindung befähigtes Strukturelement (B). Dieses Strukturelement kann jedes beliebige polare Wasserstoffatom sein, wie es beispielsweise in OH-, SH-, NH- oder PH-Bindungen vorliegt. Der Linker kann eine beliebige Kettenlänge haben. Bevorzugt ist der Linker ein (Poly)alkylenglykol, besonders bevorzugt Polyethylenglykol. Weiter bevorzugt ist ein (Poly)alkylenimin, ein (Poly)alkylenamin, ein (Poly)alkylensulfid oder ein Polyoxazilin. Der Linker kann auch mehrere der Strukturelemente (B) enthalten.

Die Kopplung der Substanzen an den Linker erfolgt nach an sich bekannten Verfahren. Hierbei können reaktive Derivate wie beispielsweise Succinimidylsuccinat, Succinimidylpropionat, Nitrophenylcarbonat, Tresylat, Epoxide, Aldehyde, Isocyanate, Maleimide und dergleichen verwendet werden. In diesem Zusammenhang wird auf den Katalog der Firma Shearwater Polymers, Inc., 2307 Spring Branch Rd., Huntsville, AL 35801 (USA) verwiesen.

Der Kunststoff ist bevorzugt ein Polymethacrylat, bevorzugt ein Polyalkyl(meth)acrylat(PAMA)-Kunststoff oder ein Polyalkyl(meth)acrylat-Mischpolymeres. Besonders bevorzugt ist der Kunststoff Polymethyl(meth)acrylat (PMMA), Polyethyl(meth)-acrylat (PEMA), Polypropyl(meth)acrylat, Polybutyl(meth)-acrylat (PBMA), Polypentyl(meth)acrylat, Polyvinylacetat, Polycyclohexyl(meth)acrylat oder Polyphenyl(meth)acrylat.

Bevorzugt sind auch Mischungen aus beliebigen Anteilen der vorstehend genannten Polyalkyl(meth)acrylate und einem oder mehreren weiteren Polymerkomponenten, beispielsweise Polystyrol, Polyacrylnitril, Polycarbonat oder Polysulfon. Beispiele für bevorzugte Mischungen sind Polymethylmethacrylat + Polystyrol, Polymethylmethacrylat + Polyacrylnitril + Methacrylat, Polymethylmethacrylat + Polyacrylnitril + Methallylsulfonat, Polymethylmethacrylat + Polyethylenpolyvinylalkohol, Polymethylmethacrylat + Polyamid, Polymethylmethacrylat + Polysulfon. Bevorzugt beträgt der Anteil an Polymethylmethacrylat in den Mischpolymeren mindestens 20 %, weiter bevorzugt sind 40% bzw. 60% des Kunststoffs aus Polymethylmethacrylat. Ebenso können Mischpolymere aus den genannten beispielhaften Polymerkomponenten hergestellt werden.

Diese Mischpolymere können nach auf dem Fachgebiet bekannten Verfahren hergestellt werden. In das Polymere können weitere Bestandteile eingelagert werden, z.B. anorganische Verbindungen wie z.B. magnetische oder paramagnetische Eisenverbindungen bzw. Eisen oder oberflächenwirksame Substanzen wie z.B. NO-abspaltende Verbindungen wie z.B. Nitroprussid-Natrium. Ferner ist auch eine Dotierung mit Metallatomen wie z.B. Nickel, Zink, Gadolinium etc. möglich. Dadurch kann das System gezielt den jeweiligen analytischen, therapeutischen oder diagnostischen Erfordernissen angepaßt werden.

Sofern das Interaktionssystem für therapeutische oder diagnostische Zwecke eingesetzt wird, ist darauf zu achten, daß der Kunststoff physiologisch verträglich ist, d.h. mit den physiologischen Flüssigkeiten keine nachteilige Wechselwirkung eingeht. Sofern das Interaktionssystem im Bereich der Lebensmittel, Kosmetika oder Bedarfsgegenstände eingesetzt wird, ist ebenfalls auf eine entsprechende Kompatibilität zu achten. Ferner kann das Interaktionssystem auch in geeignete Transportmittel z.B. Mikrosomen, Liposomen, Nanoparts etc. verpackt werden, um es leichter in den Organismus einzuschleusen.

An den Linker können beliebige Substanzen gekoppelt werden. Die gekoppelten Substanzen können gleich oder verschieden sein. Es können auch mehrere Substanzen verschiedener Art an verschiedene Linker gekoppelt werden. Bevorzugt werden pharmakologisch aktive oder physiologisch aktive Substanzen angekoppelt. Beispiele hierfür sind Proteine, Nukleinsäuren, zelluläre Signalstoffe, Partner eines biologischen oder physiologischen Affinitätspaares, wie beispielsweise DNA/DNA bindendes Protein, DNA/komplementäre DNA, RNA/komplementäre RNA, Antigen/Antikörper, Streptavidin/Biotin, Strep-TAG/künstliches Peptid, Lektin/Kohlenhydrat oder Marker für eine biologische Substanz/biologische Substanz. Besonders bevorzugt ist das Protein ein Enzym, z.B. eine Lipase, eine Esterase, eine Transferase, ein Antigen, ein Antikörper, ein Tumormarker, ein Oberflächenantigen, ein Ligand, ein Rezeptor, ein oberflächenaktives Zellfragment von Bakterien oder Viren oder ein Immunbotenstoff, wie z.B. Zytokine, Interleukine, Wachstumsfaktoren, koloniebildende Faktoren, Tumornekrosefaktoren, Apoptose-induzierende Proteine bzw. deren spezifische Antikörper oder Therapeutika. Der Marker ist beispielsweise eine fluoreszierende oder chemilumineszierende Substanz, ein EST (Expression Sequence Tag; Expressionssequenz-Code) oder ein Enzym. Die pharmakologisch wirksame Substanz ist beispielweise ein Gerinnungshemmstoff, ein stoffwechselaktives Enzym oder ein synthetischer Arzneistoff, wie beispielwese ein Antibiotikum, ein Antitumormittel oder ein Enzyminhibitor. Ferner können auch Substanzen gekoppelt werden, die mit anorganischen Ionen, wie beispielsweise Nickel, Zink, Gadolinium, Lanthan oder Gold reagieren. Beispiele hierfür sind entsprechende Komplexbildner. An einen Linker gekoppelte Substanzen besitzen ferner den Vorteil, daß ihre Ausscheidung aus dem Organismus möglich ist.

Ein derartiges Interaktionssystem erlaubt nun vielfältige Anwendungsmöglichkeiten. Es eignet sich insbesondere zur Präsentation von an den Linker gekoppelten Substanzen in einer Flüssigkeit, wobei die jeweilige Zielsubstanz gebunden, gespalten oder in sonstiger Weise modifiziert wird, bzw. zur Entfernung von mit dem Linker gekoppelten Substanzen aus einer Flüssigkeit. Ein bevorzugtes Anwendungsgebiet ist die Überwachung der Diagnose bzw. Therapie von Stoffwechselerkrankungen, Gerinnungsstörungen, viralen, bakteriellen, mykotischen oder parasitären Infektionen oder malignen Erkrankungen. Aus derartigen Interaktionssystemen lassen sich gebrauchsfertige Kits für spezielle Anwendungen sowie Vorrichtungen in entsprechenden Gehäusen und mit entsprechender apparativer Ausstattung zusammenstellen.

Obwohl das molekulare Prinzip der Wechselwirkung zwischen Strukturelement (A) und Linker bisher noch nicht untersucht ist, wird anhand der Wechselwirkung von Polyalkylenglykol und Polyalkyl(meth)acrylat folgender hypothetischer Bindungsmechanismus vorgeschlagen, der jedoch die Erfindung in keiner Weise beschränken soll. Der Carbonyl-Sauerstoff an den Poly(meth)acrylat-Seitenketten ist aufgrund der Ladungsverteilung der Kohlenwasserstoffkettenstrukturen stark negativ geladen. Dieser Sauerstoff kann also entsprechende Wasserstoffbrückenbindungen bzw. elektronische Interaktionen mit der Polyalkylenglykolkette herstellen. Die Interaktionen können auch den Sauerstoff des Polyalkylenglykols betreffen, der durch die -C-O-C-Konfiguration leicht elektronenpositiv geladen ist. Es kommt mit großer Wahrscheinlichkeit zu einer Interaktion zwischen diesen beiden unterschiedlich geladenen Sauerstoffspezies im Poly(meth)acrylat und Polyalkylenglykol, so daß die PEG-Kette über eine gewisse Strecke von mehreren C-C-O-C-C-O-Kettengliedern an die PMMA-Oberfläche gebunden wird.

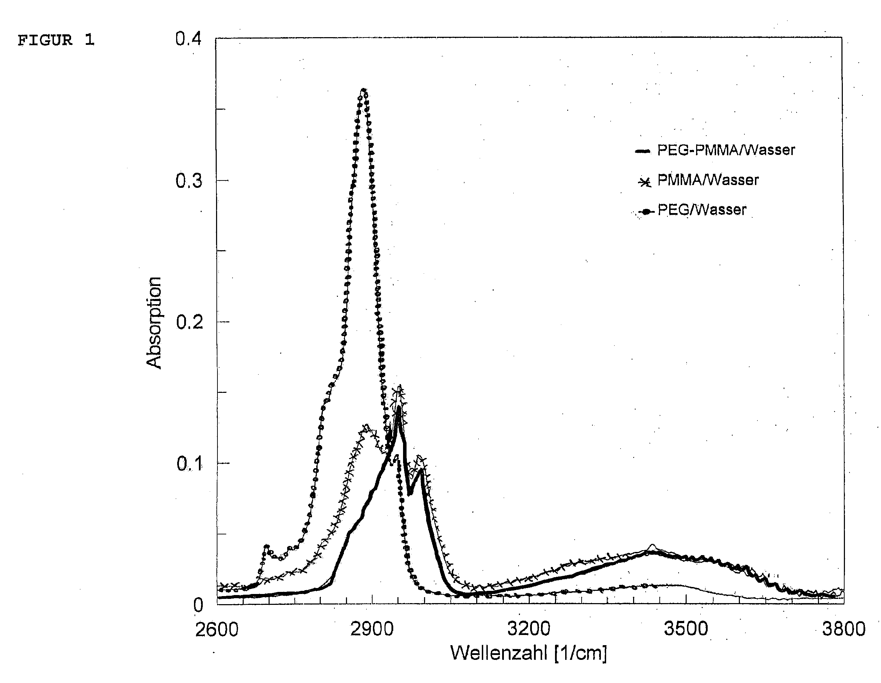

In diesem Zusammenhang wird auf das beigefügte IR-Spektrum der Figur 1 verwiesen. Diese Figur zeigt das IR-Spektrum von Polymethylmethacrylat, Polyethylenglykol (5000) und PMMA-PEG-Interaktionspartikel.

Aus der dargestellten Abbildung ist erkennbar, daß durch eine gewichtete Addition aus den PMMA- und PEG-Spektren keine Identität mit den Einzelspektren möglich ist. Besonders bemerkenswert ist die Intensitätsabnahme der symmetrischen CH₂ und CH₃-Valenzschwingung unterhalb von 2900 cm⁻¹ gegenüber dem freien PMMA. Das spricht dafür, daß durch die Adsorption von PEG und PMMA eine bestimmte neue Konformation erzwungen wurde. Als wahrscheinlichste Wechselwirkung aus der PMMA- und PEG-Interaktion sind anzunehmen:
1. Estergruppe (PMMA)..H.-O-H..Ether (PEG)
2. Estergruppe (PMMA)..H-C(Methylengruppe, PEG)
3. Carboxylgruppe (PMMA) C-O-H.. Ethergruppe (PEG) (wenige Gruppen)
4. Endgruppe (PEG)C-O-H. Estergruppe (PMMA).

Diese Zuordnungen sind alle mit den OH-Valenzschwingungen, die in den PEG-PMMA-Komplexen auftreten, konform.

Das aufgefundene Prinzip der überraschenden Bindung von mit einem Linker mit einem zur Wasserstoffbrückenbindung fähigen Strukturelement gekoppelten Substanzen an Oberflächen mit dem Strukturelement (A) läßt nun verschiedene praktische Anwendungsmöglichkeiten zu. Zum einen können derartige Oberflächen als funktionelles Antidot für spezielle Wirkstoffe eingesetzt werden. Zum anderen können derartige Oberflächen sehr leicht mit gekoppelten Wirksubstanzen beschichtet werden und somit z.B. zur in-vivo-Blutreinigung oder Plasmabehandlung verwendet werden. Von essentieller Bedeutung ist jedoch, daß dieses Interaktionsprinzip getrennt vorbereitet werden kann (Sterilisierung, Sterilfiltration u.a.) und erst danach zusammengeführt zu werden braucht. Diese Oberflächenstrukturen können Bestandteil eines extrakorporalen therapeutischen Systems sein. Ferner können gekoppelte Wirksubstanzen nach ihrer Reaktion im Organismus, bevorzugt im Blut, mit Hilfe einer derartigen Oberfläche schonend wieder aus dem Organismus entfernt werden. Dadurch können z.B. gefährlich hohe Blutspiegel von PEG-Hirudin antagonisiert werden, aber auch pegylierte Antikörper oder pegylierte Proteinwirkstoffe, die für eine definierte Zeit im Blut zur Anwendung kamen, wieder quantitativ entfernt werden (zeitlimitierte Wirkstoffanwendung). Weiterhin können z.B. PMMA-Kapillardialysatoren mittels Polyethylenglykol-gekoppeltem Hirudin oder anderen direkten Antithrombinen (z.B. Argatroban) lokal antikoaguliert werden (die Patienten brauchen dann kein systemisches Antikoagulans), aber auch durch Coatierung von entsprechenden Kapillardialysatoren mit PEG-Antigenen oder PEG-Antikörpern bzw. PEG-Wirksubstanzen zur spezifischen Interaktion mit toxischen oder krankheitsauslösenden bzw. krankheitsunterhaltenden Blutbestandteilen oder Stoffwechselprodukten befähigt werden.

Ferner kann der zur Interaktion befähigte Kunststoff auch Teil von Oberflächen von Mikrotiterplatten, Küvetten, Kontaktlinsen sein. Ferner ist es auch möglich, den Linker als solchen ohne angekoppelte Substanz zur Absättigung entsprechender freier Bindungsstellen auf dem zur Interaktion befähigten Kunststoff zu verwenden.

Für eine große Anzahl von Medikamenten wäre es vorteilhaft, ausschließlich in der Blutzirkulation zur Wirkung zu gelangen. Hierzu müßte das Molekulargewicht der Substanzen so vergrößert werden, daß sie nicht in die Flüssigkeitsräume außerhalb der Blutgefäße eindringen können. Diese molekulargewichtsvergrößerten Substanzen sind aber in der praktischen Medizin nicht verwendbar, weil dann die Elimination dieser Stoffe aus dem Körper heraus über die Nierenausscheidungswege nicht mehr möglich ist. Die "Nierenschwelle" ist in einem Molekulargewichtsbereich, der unterhalb der Größe dieser Substanzen liegt, die eine Verteilung außerhalb des Blutkreislaufs verhindert. Die ausschließliche Wirkung von Medikamenten in der Blutzirkulation ist daher ein wichtiges Problem, das bisher ungelöst ist. Erst die Möglichkeit des Herausfischens von PEG-gekoppelten Wirkstoffen (Stand der Technik) in einem definierten Molekulargewichtsbereich mit Hilfe von z.B. Polymethylmethacrylat-Oberflächenstrukturen läßt derartige Medikamente in der praktischen Medizin überhaupt erst anwendbar machen.

Bevorzugt besitzt die aktive Oberfläche eine bestimmte vergrößerte Oberflächenstruktur, z.B. Poren, die durch die Art und Weise des Spinnprozesses erzeugt werden können. Hier werden z.B. in das erhitzte Polymerisat "Fremdsubstanzanteile" zugegeben, z.B. Glycerin, welches sich nicht mit dem erhitzten, flüssigen Polymeren mischen läßt. Es entstehen Tropfen oder Störstellen in der Hohlfaserverspinnung, die dann mit geeigneten Lösungsmitteln, entweder Wasser oder organische Mittel, aus der Membranstruktur herausgewaschen werden. Hierdurch entstehen die vorteilhaften Porositäten der Membranen. Für die Mikro- und Makropartikel, die für eine weitere Anwendungsform geeignet sind, wird diese Porosität, die große "innere" Oberfläche der Partikel, in ganz ähnlicher Weise erzeugt. Auch hier werden durch Einlagerung von Fremdstrukturen, z.B. Glycerin, Raumstrukturen erzeugt, und dann nach den an sich bekannten Sprüh- oder Tropf-Polymerisationsmethoden die entsprechenden eingearbeiteten Substanzen herausgelöst. Die Polymerstruktur wird dadurch in charakteristischer Weise aufgelockert und verändert. Diese Porosität, die "innere Oberfläche" der Membranen bzw. Partikel, ist für die Bindung gekoppelter Substanzen von essentieller Bedeutung.

Die beigefügten Figuren erläutern die Erfindung näher.

Die Figur 1 zeigt IR-Spektren von Polymethylmethacrylat, Polyethylenglykol (5000) und PMMA-PEG-Interaktionspartikel.

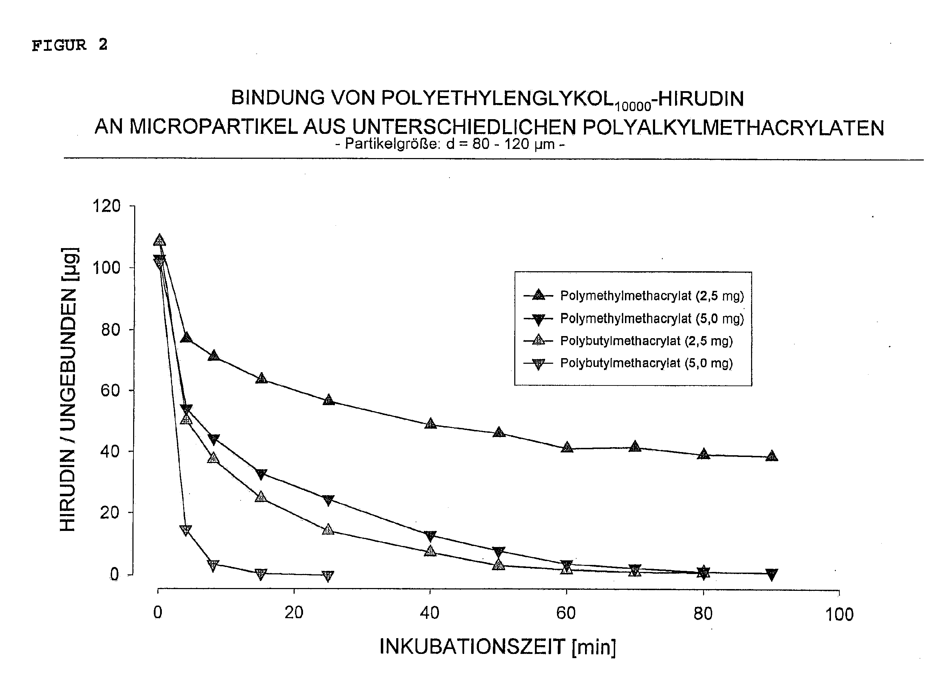

Die Figur 2 zeigt die Bindung von Polyethylenglykol₁₀₀₀₀-Hirudin an Mikropartikel aus unterschiedlichen Polyalkylmethacrylaten.

Die Figur 3 zeigt die Bindung von Thrombin an einen mit synthetischem PEG_{10kD}-Thrombininhibitor beladenen PMMA-Dialysator während wiederholter Zirkulationen von Thrombinlösungen.

Die Figur 4 zeigt die Bindung eines synthetischen PEG_{10kD}-Thrombininhibitors an einen PMMA-Dialysator während 30minütiger Zirkulationen.

Die Erfindung wird nun anhand von Polyalkylenglykol-gekoppelten Wirksubstanzen an Poly(meth)acrylatoberflächen näher erläutert.

### 1) Schlauchsysteme

Die kommerziell erhältlichen Polymethylmethacrylat-Dialysatoren, z.B. der Firma Toray, bevorzugt High-Flux-Systeme, z.B. der BK-Serie, oder Low-Flux-Dialysatoren, z.B. der B2-Serie, werden an den beiden Endseiten aufgetrennt, und die Kapillarschlauchbündel(n=60) isoliert. Nach Zuschnitt von drei Faserbündeln auf 12,7 cm Länge werden diese in einen 1,4 cm-Durchmesser-Polyethylenschlauch eingezogen. Zwischen die an beiden offenen Endseiten etwa um einen halben Zentimeter herausstehenden Kapillardialysatorbündel wird Silikon (A-Sil 2002) so eingepreßt, daß die 8-10 mm starke Vergußmasse einen totalen dichten Verschluß der offenen Enden bewerkstelligt. Nach Polymerisierung und Aushärtung der Vergußmasse werden die überstehenden Kapillarschläuche bündig mit einem scharfen Skalpell abgeschnitten, und die beiden Schlauchsystemenden werden jeweils mit Polyethylenkathetermaterial verbunden, womit eine entsprechende pumpenbetriebene Spülung der Kapillarstrecken ermöglicht wird. Die Systeme werden mit Kochsalzlösung komplett und luftblasenfrei gefüllt. In dieser Art und Weise sind diese Kapillarschlauchsysteme bei 4°C über Wochen zu lagern und anzuwenden.

### 2) Blutfilter

In 0,7-cm-Durchmesser-Polyethylenschlauchmaterial mit stärkerer Wandung werden Scheiben von Blutgasfiltermaterial aus Infusionssystemen gestanzt und diese mit einer thermoplastischen Klebmasse auf die untere Öffnung von 3 cm langen Schlauchstücken aufgeklebt. Die Kartusche wird mit porösen PMMA-Partikeln in der bevorzugten Sortierungsgröße zwischen 80 und 120 *µ*m gefüllt und auf der oberen Öffnung ebenfalls mit dem Blutsiebmaterial verklebt. Danach werden elastische Silikonkautschukschläuche straff aufgesetzt und mit dem Material mittels thermoplastischer Klebung verbunden und an Supportschläuche angeschlossen. Nach derselben Anordnung lassen sich auch Mikroblutfilter konstruieren, die aus 0,2-mm-Durchmesser-PE-Schläuchen und 2 cm Länge bestehen. Das mit Mikropartikeln zu füllende Volumen ist dann hierbei etwa auf ein Hundertstel reduziert. Diese Schlauchsysteme sind von der Größe und der Oberflächenleistung auch für einen intravasalen Einsatz geeignet.

Zur Anwendung können diese manufakturierten mikrokapillären oder mikrokolumnen Module mittels ETO oder γ-Strahlen sterilisiert werden. Auch eine Dampfsterilisierung ist für dieses Material möglich. Die Polyalkylenglykol-gekoppelten Wirkstoffe werden erst kurz vor der Anwendung des entsprechenden Systems auf die Oberfläche der Poly(meth)acrylat-Strukturen aufgebracht. Das Vorgehen ist folgendermaßen: Die Poly-(meth)acrylat-Trägermodule werden vor der Verwendung für etwa 10 min mit steriler physiologischer Kochsalzlösung gespült, um toxische Produkte, die während des Produktions-oder Sterilisationsprozesses entstanden sind, aus den Systemen herauszuspülen (Standardtechnik bei allen externen Therapiesystemen, wie Hämodialysatoren oder Oxygenatoren in der Medizin). Nach diesem Reinigungsprozeß werden die aufzubringenden, z.B. PEG-gekoppelten Substanzen in einem Rezirkulationsmodus auf die Oberfläche der Module aufgetragen, indem die Systeme für 5-10 min mit der PEG-Wirkstofflösung geprimt werden. Es ist möglich, aufgrund der großen Bindungskapazität mehrere verschiedene PEG-gekoppelte Wirksubstanzen entweder nacheinander oder miteinander gemischt auf die Poly-(meth)acrylat-Struktur aufzubringen. Es konnte in den Versuchen zweifelsfrei geklärt werden, daß kein Konkurrenzverhalten der Substanzen untereinander aufgrund besonderer chemischer Strukturen zur Poly(meth)acrylat-Oberfläche besteht. Nach Abschluß des Primingvorgangs-werden die Filter nochmals mit Kochsalz für 1-2 min gespült, um Reste der nicht komplett gebundenen PEG-Wirkstoffe aus dem System zu entfernen. Danach wird das externe therapeutische System mit Hilfe der konventionellen Ein- oder Zweinadeltechnik veno-venös bzw. arterio-venös angeschlossen. Die veno-venöse passive Applikation hat den Vorteil, daß man mit Hilfe der Miniaturblutpumpen, die mit in den extrakorporalen Kreislauf eingebracht werden, eine genaue Steuerung des Blutflusses durch das System gewährleisten kann. Die Wirksamkeit des spezifischen Substrat-"Fishings" wird anhand des Verschwindens der Zielpathogene überprüft. Hierzu existieren nahezu für alle verwendeten Wirkstoffe entsprechende Nachweismethoden.

Die Anwenderkits der externen therapeutischen Systeme sind dergestalt, daß verschiedene Größen der Blutfilter zur Auswahl stehen, um diese der notwendigen Menge von geprimter Substanz anzupassen. Hierzu gibt es ein Umrechnungsmaß, welches aus der präklinischen Erprobung derartiger Systeme bekannt ist. Der behandelnde Arzt kann aus einem Reservoir unterschiedlichster pegylierter Wirkstoffe und verschiedenster Blutfilter nach einer Art "Baukastenprinzip" das für seinen Patienten hinsichtlich einzusetzender Therapeutika und Größe des Systems individuell erforderliche therapeutische Prinzip zusammenstellen. Derartige Kits enthalten gegebenenfalls geeignete Reagentien, Pufferlösungen, Reaktionsgefäße und dergleichen. Das Kit kann auch in einer geeigneten Verpakkung vorliegen.

Solche Anwendungskits können in gleicher Weise auch als biochemische Reinigungssysteme zusammengestellt werden. Die Modulgröße wird der entsprechenden präparativen Verwendung angepaßt. Für die biochemischen präparativen Fragestellungen, für in vitro-Untersuchungen, ist nicht nur das Säulenverfahren, sondern auch das Batchverfahren zu verwenden. Die entsprechenden Mikro- oder Makropartikel werden in einem "Batch"-Volumen intensiv gemischt, und nach Priming mit den polyalkylierten Wirkstoffen und Zentrifugation des Überstandes werden diese beladenen Partikel noch zweimal mit Kochsalz gewaschen und danach mit der entsprechenden Präparationslösung versetzt. Durch das Herausfischen der "passenden" Reaktionspartner kann dann eine Trennung zwischen Antigenen und Antikörpern bzw. chemisch interaktiven Substanzen erfolgen, und dadurch ist z.B. eine präparative Affinitätsreinigung mit nahezu 100% Effizienz möglich.

Nachstehend ist eine beispielhafte Liste von Anwendungsmöglichkeiten für die oben genannten Systeme angegeben.
A) Entfernung von Substanzen aus dem Blut:
   1. Zur Verwendung von spezifischen Antigen-Antikörper-Reaktionen werden die vorhandenen monoklonalen oder polyklonalen Antikörper mit Polyethylenglykol gekoppelt und diese PEG-Antikörper dann auf die PMMA-Oberfläche gebracht. Die möglichst spezifische Erkennung der Epitope des zu entfernenden Stoffs ist von großer Wichtigkeit für die schnelle und feste Bindung an die vorbereitete Oberfläche. Nach oben beschriebenem Vorgehen werden die PEG-Antikörper auf die gewählte Blutfiltergröße aufgeprimt, und durch den Zusatz eines pegylierten Antithrombins, z.B. PEG-Hirudin, ist eine lokale Antikoagulation der Systeme jeweils anzustreben. Der Vorteil der lokalen antikoagulativen Wirkung mit Hilfe von pegylierten Antithrombinen besteht vor allem darin, daß der zu behandelnde Patient während der Behandlung mit den externen therapeutischen Systemen nicht systemisch antikoaguliert werden muß.
   2. Zur Behandlung von Hemmkörper-Hämophilie, einer schweren Autoimmunkrankheit, wird humaner Faktor VIII (antihämophiles Globulin) mit den bekannten Methoden an Polyethylenglykol gekoppelt und dann auf einen Polyalkylacrylatträger geeigneter Größe geprimt. Die nach dieser Methode hergestellten Hemmkörper-Hämophilie-Module werden in regelmäßigen Abständen bei den betroffenen Patienten als externes therapeutisches System angewendet. Die zeitlichen Intervalle dieser Behandlung richten sich nach dem meßbaren "Verbrauch" des Faktor VIII im Patienten. Der Faktor-VIII-Spiegel sollte nicht unter 25-30 % des normalen Wertes sinken. Zwischen den Anwendungsintervallen sind die Patienten ohne jegliche Blutungsgefahr bzw. Blutungstendenz, so daß diese Therapieform als eine Kausaltherapie zu bezeichnen ist.
   3. Antimikrobielle Behandlungsmöglichkeiten: Mit den Methoden der molekularen Medizin ist es möglich, gegen nahezu alle Viren, die proteinhaltige Hüllen haben, gegen Bakterien, aber auch gegen Pilze monoklonale bzw. polyklonale Antikörper herzustellen. Diese gegen mikrobenspezifische Proteine gerichteten Antikörper lassen sich nach Kopplung mit Polyethylenglykol geeigneter Größe (vorzugsweise 5-10 kDa) auf die Wirkstoffträger aus Polyalkylacrylat primen. Durch die Behandlung der Patienten mit derartigen externen therapeutischen Systemen ist eine Entfernung der krankheitsauslösenden und -unterhaltenden Keime aus der Blutbahn möglich. Speziell ist diese Methode exzellent geeignet, z.B. auch Malaria zu behandeln. Durch den zyklischen Einsatz von spezifischen Wirkstoffträgern ist die Erkrankung zu unterbrechen und eine rasche Heilung des Patienten zu erzielen.
   4. Herstellung von lokal antikoagulativen Hämodialyse- und Hämofiltrationssystemen: Bei der Verwendung von Polymethylmethacrylat-Hämodialysatoren oder -Hämofiltrationsmodulen (Produkte der Firma Toray, Japan) werden vor dem Einsatz nach Spülung der Systeme diese Module mit Polyethylenglykol-Hirudinlösung behandelt. Durch Rezirkulation von PEG-Hirudin ist ein antikoagulativer Effekt an der Membranoberfläche zu erreichen. Die Systeme sind je nach Länge der Anwendung mit variablen Mengen von PEG-Hirudin zu behandeln (5-50 mg).
   5. Herstellung von lokal antikoagulierten Blutschlauchsystemen: Durch Verwendung gebräuchlicher Blutschlauchsysteme (PS, PE, PP u.a.), bei deren Herstellung mindestens 20%, besser 50% Polyalkylmethacrylate, bevorzugt Polybutylmethacrylat zugesetzt wurde, ist ein Priming mit PEG-Hirudin oder PEG-gekoppelten synthetischen Thrombininhibitoren möglich. Dadurch ist auch eine lokale Antikoagulation im gesamten Verlauf der Blutschlauchsysteme, sowohl arteriell als auch venös, möglich.
B) Aufbringen von Enzymen, die bestimmte Blutbestandteile bzw. Proteine beeinflussen:
   Hier kommen z.B. folgende Substanzen in Betracht: 1. Enzyme, die direkt oder indirekt in Steuermechanismen des Körpers eingebunden sind, z.B. spezielle Gerinnungsfaktoren aktivieren oder inhibieren. Durch die PEG-Kopplung von z.B. Protac, einem Schlangengiftenzym, welches hochspezifisch Protein C aktiviert, könnte ein vollkommen neuer Weg der langzeitigen Thromboseprophylaxe ohne Blutungsneigung oder andere Nebenwirkungen i.S. der bisherigen therapeutisch verwendeten Substanzen eröffnet werden. Das Enzym Protac aktiviert Protein C zu Protein C_{A}, das in die Gerinnung durch Hemmung der aktivierten Faktoren V und VIII eingreift. Beide Faktoren werden durch Thrombin aktiviert und haben einen positiven Feedbackmechanismus auf die Thrombinaktivierung selbst, so daß sich daraus ein Potenzierungskreislauf ausbildet, der sich durch die Inhibierung dieser beiden Akzelleratorfaktoren schlagartig unterbrechen läßt (klinische Korrelate sind Protein-C-Mangel-Zustände mit schweren thromboembolischen Erkrankungen). Aber auch zuckerspiegelregulierende Enzyme wie Glucose-Dehydrogenase und andere Enzyme könnten im Blut pathologische oder pathophysiologische Veränderungen i.S. einer Stoffwechselerkrankung regulieren (Hypercholesterinämie, Hyperlipidämie, Diabetes mellitus Typ II oder bestimmte Speicherkrankheiten). Die Beeinflussung von endokrinologisch bedingten Stoffwechselstörungen ist hier eine mögliche Anwendungsform.
C) Aufbringung von für Vitalfunktionen wichtigen Substanzen
   1) Kunstblut
      Auch besteht die Möglichkeit, künstliche "rote Blutkörperchen" bereitzustellen. Ein PEG-Hämoglobin, welches z.B. aus Rinderblut gewonnen wird, ist derzeit als "Kunstblutkomponente" in der klinischen Prüfung ("Oxyglobin"). PEG-Hämoglobine sollten auf entsprechende größensortierte paramagnetische Polymethylmethacrylat- oder Polyalkylmethacrylat-Partikel aufgebracht werden und dann entsprechend angewendet werden. Idealerweise besitzen die Partikel das gleiche spezifische Gewicht wie Blut. Bevorzugt werden daher Polyalkylmethacrylat-Polystyrol-Copolymere in einem 50/50-Mischungsverhältnis, bevorzugter in einem 30/70-Mischungsverhältnis benutzt. Dazu wird z.B. 1 g monodisperse, poröse Polymethylmethacrylatpartikel (oder PMMA-Polystyrol) (d = 6 *µ*m) mit 250 mg PEG-gekoppelten Hämoglobins (z.B. Versuchspräparat der Firma Enzon) geprimt. Die Gewichtsangabe bezieht sich auf den Hämoglobinanteil. Die hierzu verwendeten Polymethylmethacrylatpartikel (PMMA-Polystyrol) sind speziell zubereitet worden, indem im Produktionsprozeß während der Polymerisierung Fe₂O₃ in feindisperser Form in die Polymerisierungsmasse eingebracht wurde. Diese Fe₂O₃-haltigen Partikel sind paramagnetisch und lassen sich dann auf die vorstehend beschriebene Art und Weise aus der Zirkulation wieder entfernen.
      Derartige magnetisch aktive Partikel sind in vielfältiger Form für eine direkte zirkulative Anwendung im Blut geeignet. Neben dem oben beschriebenen PEG-Hämoglobin können auch NO-Donatoren (spezifische Hemmung der Blutplättchenaktivierung), aber auch spezifische Interaktionspartner für Zellen, vorzugsweise Tumorzellen, aufgebunden werden. Bei der Behandlung von Tumoren kann eine "Fixierung" der Antitumorwirkstoff-tragenden Partikel mittels Magnetfeldern im Tumorbereich realisiert werden.
      Die Hämoglobinpartikel haben eine hohe Sauerstoffbindungsfunktion und können den Sauerstofftransport in der gesamten Blutzirkulation in ähnlicher Weise, wie es die Funktion der roten Blutkörperchen ist, übernehmen. Dadurch eignen sich diese Hämoglobin-Mikropartikel für die Katastrophenmedizin als universelle Sauerstoffträgersysteme.
      Von besonderer Bedeutung ist, daß der Sauerstofftransport als die wesentlichste gestörte Vitalfunktion nach großen Blutverlusten sehr schnell wiederhergestellt wird. Am Ende der mehrtägigen Behandlung, wenn der betroffene Organismus wieder entsprechende Blutkörperchen nachsynthetisiert hat, können die Partikel quantitativ aus der Zirkulation mit Hilfe von Magnetrückhaltesystemen entfernt werden. Diese Magnetrückhaltesysteme sind z.B. mikrokapillare ETS, die von einem Permanentmagnetring umgeben sind. Die magnetischen Partikel werden durch Anheftung an die Hohlfasern des ETS aus der Zirkulation entfernt.
   2) Zellwachstumsfaktoren
   Das beschriebene Grundprinzip der Anheftung von Wirkstoffen auf spezifischen Polymermaterialien eignet sich auch zur Beschichtung von anderen Kunststoffoberflächen, von Zellseparatoren bzw. Zellzuchtmaterialien, bei denen ein verstärkter Wachstumsschub dadurch erreicht wird, daß durch Aufkleben von dünnen Schichten feinster Mikropartikel aus PMMA eine Aufbringung von Zellwachstumsfaktoren (z.B. pegylierter BMP-2 oder BMP-4) möglich wird. Das kann aber auch ausgenutzt werden, um Gefäßprothesen mit Wachstumsfaktoren so zu beschichten, daß Endothelzellen sehr schnell und intensiv auf der Blutseite der Gefäßprothesen angesiedelt werden und dort eine Naturalisierung ("Endothelialisierung") der Gefäßprothese einleiten. Durch die spezifische Dotierung der Gewebeseite der Prothesen zum Zweck des Einwachsens von Bindegewebszellen und glatten Muskelzellen, z.B. durch das Aufbringen von basischem Fibroblasten-Wachstumsfaktor (bFGF) ist auch eine Naturalisierung des gesamten Prothesenmaterials möglich. Die Beschichtung von Gelenkprothesenmaterial mit pegyliertem rBMP-2 oder rBMP-4, zwei rekombinant hergestellten Osteoblastenwachstumsfaktoren, oder die Beimischung von porösen Mikropartikeln aus PAMA mit rBMP-2 oder rBMP-4 in Knochenzementen läßt die Einwachsungsphase stark verkürzen. So werden weit verbesserte Fixierungsergebnisse erreicht.
D) Separationstechniken von Mikropartikeln für Viren und Bakterien bzw. deren Stoffwechselprodukte in Blut- und Plasmaspenden
   Durch die Behandlung des Plasmas mit spezifischen PMMA-PEG-Antikörpern gegen Hüllproteine oder DNA von Viren ist es möglich, sehr schonend humane Plasmaproben zu reinigen. Vor allem für Viren mit spezifischen Proteinen (Hepatitis B-Virus-Protein E₁ und E₂, AIDS-Virus-Proteine) ist eine quantitative Entfernung möglich. Das kann sowohl im Batch- als auch im Säulenverfahren erfolgen.
E) Verwendung von PMMA-PEG-Partikeln zur Entfernung von unerwünschten Substanzen aus Lebensmitteln, beispielsweise zur Entalkoholisierung von alkoholischen Getränken wie Bier, Wein etc., Präsentation von speziellen Lipasen bzw. Enzymen, die übermäßige Nahrungsfette in nicht resorbierbare Derivate abbauen bzw. entsprechende Verbindungen an ihre Oberfläche binden, Entfernung von Cholesterin, gezielte Entfernung von bestimmten Eiweißbestandteilen bei bestimmten Stoffwechselerkrankungen (z.B. Gluten bei Zoeliakie), Entfernung von speziellen Kohlenhydraten bei Diabetes etc.)
   Auf dieser Basis lassen sich Design-Food und Diätprodukte für bestimmte Ernährungsbesonderheiten herstellen, indem entsprechend beschichtete erfindungsgemäße Mikropartikel dem Produkt bereits hinzugefügt werden, oder das Produkt vorher entsprechend behandelt wurde. Es ist auch möglich, entsprechende Mikropartikel separat vor, während und nach Genuß entsprechender Speisen einzunehmen. Die Mikropartikel können zu diesem Zweck in magensaft- bzw. darmsaftresistente Tabletten oder Kapseln konfektioniert werden.
F) Installation von Mikroblutfiltern in die Blutbahn mit Hilfe der sogenannten Venen"schirm"technik, wie sie bei tiefen Bein- und Beckenvenenthrombosen heute schon über die invasive Radiologie angewendet wird. Hierbei wird ein sogenannter Cava-Schirm, der die Form eines Drahtkäfigs hat, aufgespannt und in der Vena cava installiert, um größere thrombotische Massen an deren Abschwemmen in die Lungenstrombahn zu hindern. Diese Cava-Schirm-Technik könnte auch als Halterung bzw. Trägermaterial für Mikroblutfilter verwendet werden. Zur Anwendung kommen dieselben Beschichtungsmaterialien, wie sie oben beschrieben sind. Ein entsprechendes System sollte aber hier mehr die Form eines flachen Blutfilters mit möglichst großer Austauschfläche haben.
G) Orale Anwendung von partikulären Wirkstoffträgern:
   Hier ergeben sich zwei unterschiedliche Möglichkeiten:
      1) Substitution von gestörten Verdauungsfunktionen:

      Ähnlich der in Entwicklung befindlichen Deliverysysteme für Verdauungsenzyme können auch bestimmte erfindungsgemäße Partikel, die einen permanenten Verdauungsenzym-Überzug erhalten, verwendet werden. Diese Partikel können wesentliche Verdauungsfunktionen während ihrer Passage im Magen-Darm-Kanal innehaben. Eine permanente Instillation von verdauungsfördernden Darmsonden ist möglich, wenn an einer entsprechenden fibrillären oder ähnlichen Struktur die Enzyme im Dünndarm auf einer bestimmten Polyalkylacrylat-Matrix verwendet werden. Eine permanente Magen-Verweilsonde in Form eines strukturierten Kunststoff-Käfigs wird z.B. mit filamentären PMMA-Strukturen ausgerüstet, an denen auf einer Strecke von 30-50 cm die Enzyme permanent über die PMMA-PEG-Kopplung angebracht sind. Sie werden mit dem Nahrungsbrei in den Dünndarm eingeschwemmt und haben an einem Ende noch festen Kontakt zur Magenhalterung. Die entsprechenden Enzyme könnten für eine längere Zeit im Magen-Darm-Kanal die Verdauungsfunktion imitieren, sie treten dann in Aktion, wenn entsprechende Nahrungsbestandteile die fibrillären Strukturen passieren.
         2) Korpuskuläre bzw. partikuläre Enzymträger, die spezielle Anteile oder bestimmte Nahrungsbestandteile metabolisieren, mit dem Ziel, daß diese in ein nicht mehr resorptionsfähiges chemisches Derivat verändert werden. Hierzu eignen sich pflanzliche Enzyme, die in der Lage sind, Cholesterin und andere fett- und energiereiche Verbindungen der Nahrung zu verändern. Eine Bindung von Cholesterin und fettresorbierender Gallensäuren an entsprechende Bindungspartner an der Oberfläche dieser Magen-Darm-Partikel ist ebenfalls ein möglicher Weg. Zur speziellen Anwendung eignen sich auch erfindungsgemäße Partikel mit hoher Konzentration von Alkoholdehydrogenase, um unerwünschte Mengen von Alkohol bereits im Magen-Darm-Kanal abzubauen.
      Eine spezielle Anwendung ist beispielsweise die Verwendung pegylierter monoklonaler Antikörper gegen den Tumornekrosefaktor (TNF). Der monoklonale Antikörper gegen den Tumornekrosefaktor (MAK-195) ist ein Versuchspräparat der Firma Knoll und wird augenblicklich in klinischen Studien bei Schockpatienten auf seine klinische Effizienz hin evaluiert. Dieser monoklonale Antikörper läßt sich problemlos mit PEG koppeln und auf eine entsprechende PMMA-Struktur aufbringen. Dadurch ist der monoklonale Antikörper festphasengebunden und bindet hochspezifisch ein Epitop des Tumornekrosefaktors. So können hohe und pathophysiologisch für den weiteren Verlauf einer Schockreaktion schädliche TNF-Spiegel gesenkt werden. In entsprechender Weise lassen sich monoklonale bzw. polyklonale Antikörper gegen Lipopolysaccharid-bindendes Protein (LBP) koppeln. Dieses Protein dient dem Transport bakterieller Stoffwechselprodukte der Lipopolysaccharide (Endotoxine, Lipid A) im Blut und ihrer Präsentation an die Zellen. Wird der Spiegel des Lipopolysaccharid-bindenden Proteins gesenkt, kann dadurch die Aufnahme von Endotoxinen aus dem Magen-Darm-Kanal, aber auch aus septischen Herden vollständig blockiert werden.
      Ferner ist es beispielsweise auch möglich, spezifische humane γ-Globulinfraktionen zu pegylieren und auf die PMMA-Membran zu coatieren, um Bakterien bzw. Viren direkt zu hemmen und über das temporäre extrakorporale therapeutische System aus dem Organismus zu entfernen.
      Ferner können auch Inhibitoren für aktivierte Gerinnungsfaktoren, z.B. der rekombinante TFPI, ein natürlicher Inhibitor des Faktor VII-Tissuefaktor-Komplexes, des wichtigsten Startmechanismus der Blutgerinnung, eingesetzt werden. Diese können in entsprechend pegylierter Form auf der Membran gebunden werden, um damit die Blutgerinnung in den ersten Aktivierungsmechanismen vollständig zu hemmen, ohne dabei das normale Gerinnungspotential des Organismus zu beeinflussen. Prinzipiell läßt sich jeder pathologische Mechanismus des Organismus, der zu seiner Signalübertragung den Blutweg benützt, durch ein derartiges extrakorporales therapeutisches System modifizieren.
      Die Bindungsfähigkeit derartiger oberflächendotierter Kapillardialysatoren ist erstaunlich groß; es können bis zu 1 g pegylierte Strukturen auf 1 m² Oberfläche gebunden werden. Mit Hilfe von Auswaschversuchen konnte zweifelsfrei belegt werden, daß die physikochemisch erklärbare Verbindung zwischen dem pegylierten Wirkstoff und der PAMA-Membran sehr stabil ist und ein Ablösen von der Membran sowohl durch Kochsalzspülung als auch bei Spülen mit Plasma bzw. mit Vollblut nicht möglich ist.
      Aus der molekularen Immunologie ist eine Reihe spezifischer Tumorantigene an Zelloberflächen von Tumorzellen bekannt, für die zu diagnostischen Zwecken auch entsprechende Antikörper für in-vitro-Untersuchungen zur Verfügung stehen. Deren Fremdeiweißstrukturen verbieten jedoch eine Anwendung beim Patienten. Erfindungsgemäß können nun diese Antikörper, an PEG-Linker an der Oberfläche einer PMMA-Membran gebunden, eine spezifische Interaktion mit Tumorzellen des Blutes eingehen und diese ähnlich der Hämofiltration spezifisch aus der Zirkulation entfernen. Damit wird erstmalig eine Anordnung bereitgestellt, in der mit vorhandenem Oberflächenmaterial, das bioverträglich und zugelassen ist, eine spezifische Interaktion im Blut zwischen Zellen bzw. Zellanteilen des Organismus und festphasengebundenen spezifischen Gegenspielern möglich ist.
      Die erfindungsgemäßen Interaktionssysteme lassen sich auf viele weitere therapeutische Anwendungen, insbesondere auf die in-vivo-Plasmareinigung, anwenden. Beispielsweise können Ultrafiltrationsmembranen vom Typ PMMA, auf denen spezielle PEG-gekoppelte Enzyme angebracht sind, zur Entgiftung bei nierenkranken Patienten eingesetzt werden. Ferner können durch Aufbringen von PEG-gekoppelter Urease auf PMMA-Membranen erhöhte Harnstoffwerte im Blut abgebaut werden oder durch Aufbringen PEG-gekoppelter Kreatinin-metabolisierender Enzyme eine Senkung weiterer harnpflichtiger Stickstoffprodukte veranlaßt werden.
      Bevorzugt ist die Verwendung der erfindungsgemäßen Interaktionssysteme, bevorzugt von PMMA-Membranen in Kapillardialysatoren. Der alleinige Hersteller von PMMA-Kapillardialysatoren ist die Firma Toray, Japan. Vor ihrer Anwendung am Menschen werden die Kapillardialysätormodule sowohl auf der Dialysatseite als auch auf der Blutseite intensiv gespült, auf der Blutseite mit physiologischer Kochsalzlösung, auf der Dialysatseite mit Dialysatflüssigkeit (Salzlösung), um toxische Produkte und Substanzen, die während des Produktionsprozesses mit dem System in Kontakt gekommen sind, aus dem Kapillardialysator herauszuspülen. Am Ende dieses kontinuierlichen Spülvorgangs, bei dem die Spülflüssigkeit auf beiden Seiten verworfen wird, wird für die Vorbereitung des externen therapeutischen Systems die Dialysatseite durch Blindstopfen verschlossen; auf der Blutseite wird eine Rezirkulation angeschaltet, wobei in der Vorlage von etwa 200 ml die pegylierte Auftragungssubstanz gelöst ist. Durch einen einfachen Rezyklisierungsvorgang, bei dem an dem Ausgang des Dialysators über eine Rollerpumpe die Lösung in das Lösungsreservoir zurückgepumpt wird, wird die Membran innerhalb von 5-10 min mit der pegylierten Substanz coatiert. Die PMMA-Membranen nehmen unabhängig von ihrem Cutoff (Maßzahl für die Größe der Poren in der Membran) bis zu 200 mg der aufzutragenden Substanz (= 1 g der pegylierten Wirksubstanz) pro m² Kapillaroberfläche auf. Es ist wichtig, daß die entsprechenden Bindungsstellen auf der PMMA-Membrän vollständig von den pegylierten Wirksubstanzen besetzt sind. Falls nur geringere Mengen dieser Wirksubstanzen aufgebracht werden, sollte man am Ende des Spülprozesses eine Nachspülung mit PEG-Kochsalzlösung nachlaufen lassen, um alle PEG-Bindungsstellen der Membran abzusättigen. Dadurch ist gewährleistet, daß eine zusätzliche Coatierung mit Plasmaproteinen des Blutes an den Bindungsstellen nicht mehr auftritt und damit keine Interaktion zwischen Plasmaproteinen und den präsentierten Wirksubstanzen zustandekommt (Priming).
      Die Erfindung wird nun anhand der folgenden Beispiele näher erläutert.

### Beispiel 1

### Verwendung von Polyethylenglykol-gekoppeltem Hirudin in der Dialyse

Hirudin stellt in der rekombinanten, naturidentischen Form ein hochspezifisches und effektives antithrombotisches Prinzip dar, welches in den nächsten Jahren für die suffiziente Therapie thromboembolischer Erkrankungen und deren Folgezustände zunehmendes Interesse gewinnen wird.

Erste klinische Anwendungen und große Phase III-Studien haben die Effektivität dieser neuartigen, therapeutisch wertvollen Substanz bewiesen.

Rekombinantes Hirudin kann natürlich auch zu Intoxikationen führen, entweder durch die Art des durchgeführten Therapieschemas, durch Überdosierung oder durch objektive Eliminationsschwierigkeiten, die durch plötzliche oder passagere Niereninsuffizienzen gegeben sind.

Für die Entfernung von Hirudin aus dem Organismus stehen augenblicklich in der klinischen Medizin zwei Wege offen, nämlich die Applikation eines Antidots (vgl. die deutschen Patentschriften P 42 03 965.7 und 196 25 642.9 und EP 0 625 908), sowie die rasche Entfernung des Hirudins mittels High-Flux-Dialysemembranen.

In der Klinik hat sich vor allem ein großmolekulares Derivat des Hirudins für die praktische Anwendung als geeignet erwiesen. Es handelt sich um ein Hirudin, bei dem das Molekulargewicht durch zwei 5000-D-Polyethylenglykol-Ketten vergrößert ist. Dadurch wird nach Applikation eine bis zu 4-5 mal größere Stoffmenge im Organismus und eine stark verlängerte Plasmaverweilzeit des Hirudins erreicht.

Die Möglichkeiten, dieses PEG-Hirudin bei Überdosierungen oder toxischen Blutspiegeln zu antagonisieren, stellt sich als schwierig heraus. Zwar ist für das PEG-Hirudin ebenfalls das universelle Antidotprinzip gegen Hirudine und gegen synthetische Thrombin-Inhibitoren wie oben beschrieben verwendbar, aber eine generelle Dialysierbarkeit von PEG-Hirudin ist nicht gegeben. Selbst Hämofiltrationssysteme mit extrem großem Cutoff werden von PEG-Hirudin nicht passiert. Dementsprechend ist bisher eine schnell verfügbare funktionelle Antidotformulierung nicht möglich.

Beim Vergleich zwischen Hirudin und PEG-molekulargewichtsvergrößertem Hirudin bei entsprechenden in-vitro-, aber auch in-vivo-Zirkulationen durch PMMA-Kapillardialysatoren und PMMA-Hämofiltern wurde ein gänzlich unterschiedliches Verhalten festgestellt. Während natives oder rekombinantes Hirudin sehr rasch von der Blutseite in die Dialysatseite übertritt und aus der Zirkulation verschwindet, verhält sich PEG-Hirudin total anders. Es wurde nachgewiesen, daß bei einem Kapillardialysator-Rezirkulationsmodell nach kurzer Zeit sowohl auf der Blut- als auch auf der Dialysatseite kein PEG-Hirudin in freier Form mehr vorkommt, obwohl relativ hohe Mengen von PEG-Hirudin auf der Blutseite entweder in antikoaguliertes Rinderblut oder auch in Eiweißkochsalzlösung appliziert wurden. Damit wurde nachgewiesen, daß in diesem Rezirkulationsmodell PEG-Hirudin mit hoher Geschwindigkeit aus Vollblut, Plasma oder albuminischer Rezirkulationslösung auf der PMMA-Membranoberfläche fest gebunden wird. Ein Versuch, nach Entfernung der untersuchten Zirkulationslösung mit Hilfe von Kochsalzlösungen ein Ausbluten bzw. Auswaschen des PEG-Hirudins von der Membran zu erreichen, war nicht möglich; die Bindung erwies sich durchaus als stabil und fest.

Aus diesen Untersuchungen ergibt sich eindeutig, daß damit ein funktionelles Antidotprinzip für PEG-Hirudin möglich ist. Derartige Dialysatoren können auf verschiedene Weise genutzt werden:
Man kann beispielsweise einen PMMA-Dialysator an eine normale Dialyse-Einheit anschließen und als funktionelles Antidotprinzip für PEG-Hirudin bei entsprechenden klinischen Einsätzen verwenden, oder man kann auch mit einfachen Hämofiltrationspumpsystemen, wie sie in der Intensivmedizin zur kontinuierlichen Hämoperfusion angewendet werden (siehe Beispiel), die PMMA-Dialysatoren mit verschlossener Dialysatseite betreiben, so daß eine relativ problemlose, gering volumenbelastende "Blutwäsche" des betroffenen Patienten vorgenommen werden kann. Die benötigte Blutwäschezeit selbst für hohe Konzentrationen von PEG-Hirudin im Blut wäre in diesen Fällen mit weniger als 2 h, vorzugsweise 30-45 min, anzugeben.
Zur extrakorporalen Therapie des Blutes mit Hilfe der effektiven Dialyse harnpflichtiger Stoffwechselprodukte werden möglichst gut biokompatible Membranmaterialien entwickelt, die über eine lange Zeit konstante Filterleistungen haben. Polymethylmethacrylat (PMMA) bietet eine exzellente Clearance von mittleren Molekülen und Ultrafiltrationsleistungen, die durch den Transmembrandruck exakt kontrolliert werden können.

### Beispiel 2

### Herstellung von PEG-modifiziertem Hirudin-Ziegenantikörpern

### 1) Präparation von SC-PEG

Methode nach T. Miron und M. Wilchek (Lit.: Bioconjugate Chem. 1993, 4, 568-569)

| **Materialien:** | |
|---|---|
| Aceton | wasserfrei |
| Diethylether | wasserfrei |
| 1,4-Dioxan | wasserfrei |
| Molekularsieb | 4A, Perlen 8-12 mesh |
| Phosphat-Puffer | 0,05 mol/l; pH 7,0 |
| Polyethylenglykol 6000 | MG 5000-7000; 1 mmol |
| N,N'-Disuccinimidylcarbonat | 5 mmol |
| 4-(Dimethylamino)pyridin | 5 mmol |

### Ausführung:

In 25 ml wasserfreiem Dioxan (über Molekularsieb) werden 1 mmol PEG im heißen Wasserbad gelöst. Nach Abkühlung auf Raumtemperatur wird das N,N'-Disuccinimidylcarbonat, gelöst in 10 ml Aceton, zugegeben. Anschließend läßt man langsam die 10 ml Aceton mit 5 mmol 4-(Dimethylamino)pyridin unter Rühren zufließen. Bei Raumtemperatur wird die Mischung 6 Stunden aktiviert (Magnetrührwerk). Das aktivierte PEG wird aus dieser Lösung direkt mit Diethylether gefällt. Durch eine G3-Fritte wird der voluminöse weiße Niederschlag abgesaugt. Die völlige Entfernung des Ethers erfolgt im evakuierten Exsiccator. Aufbewahrung: trocken bei 4°C.
**Effizienz: 85 %** (bezogen auf PEG)

### 2) Kopplung von SC-PEG mit Protein

### a) Präparation von PEG-modifiziertem Hirudin:

| | |
|---|---|
| **Materialien:** | Borat-Puffer pH 9,5 |
| | Phosphat-Puffer pH 7,0; 0,05 mol/l |
| | Hirudin; rekombinant |
| | SC-PEG |

### Ausführung:

150 mg PEG und 27 mg Hirudin wurden zusammen in 25 ml Borat-Puffer gelöst und 3 Stunden bei 4°C unter Rühren inkubiert. Ungebundenes PEG und Hirudin werden durch Ultrafiltration (AMICON; Membran YM 10) mit dem 6- bis 10-fachen Volumen Phosphat-Puffer entfernt. Das Retentat wird lyophilisiert und im Exsiccator bei 4°C aufbewahrt. Die einzelnen Präparationsschritte wurden durch SDS-PAGE, Gerinnungsteste (ECT), PEG-Bestimmung und Tierversuch belegt.

### b) Präparation von PEG-modifiziertem Hirudin-ZAK:

| | |
|---|---|
| **Materialien:** | Phosphat-Puffer pH 7,0; 0,05 mol/l |
| | SC-PEG |
| | Hirudin-Ziegenantikörper (Hi-ZAK) |

### Ausführung:

500 mg PEG werden in 20 ml Hi-ZAK (in Phosphat-Puffer vorliegend) gelöst und 48 Stunden bei 4°C unter Rühren inkubiert. Ungebundenes PEG und Hi-ZAK werden durch Ultrafiltration (AMICON; Membran YM 10) mit dem 6- bis 10-fachen Volumen Phosphat-Puffer entfernt. Die Aufbewahrung des Präparats erfolgte als Lösung bei 4°C.

Diese PEG-modifizierten Hirudin-Ziegenantikörper können auf eine PMMA-Membran aufgebracht werden, und damit lassen sich bei Verwendung in einem extrakorporalen therapeutischen System schonend hohe Hirudinspiegel im Blut senken.

### Beispiel 3

### "Entfernung" von PEG-Hirudin aus Blut

In dem folgenden Versuchsbeispiel soll die Effizienz der Entfernung von PEG-Hirudin aus dem Blut dargestellt werden. Zu diesem Zweck wird 250 bzw. 300 ml Rindervollblut mit 36 *µ*g/ml PEG-Hirudin antikoaguliert und in entsprechend aufbereitete und für die Anwendung zur Verfügung stehende PMMA-Dialyse-Rezirkulationssysteme eingebracht. Zu diesem Rezirkulationsmodell wird folgender Versuchsaufbau benutzt:

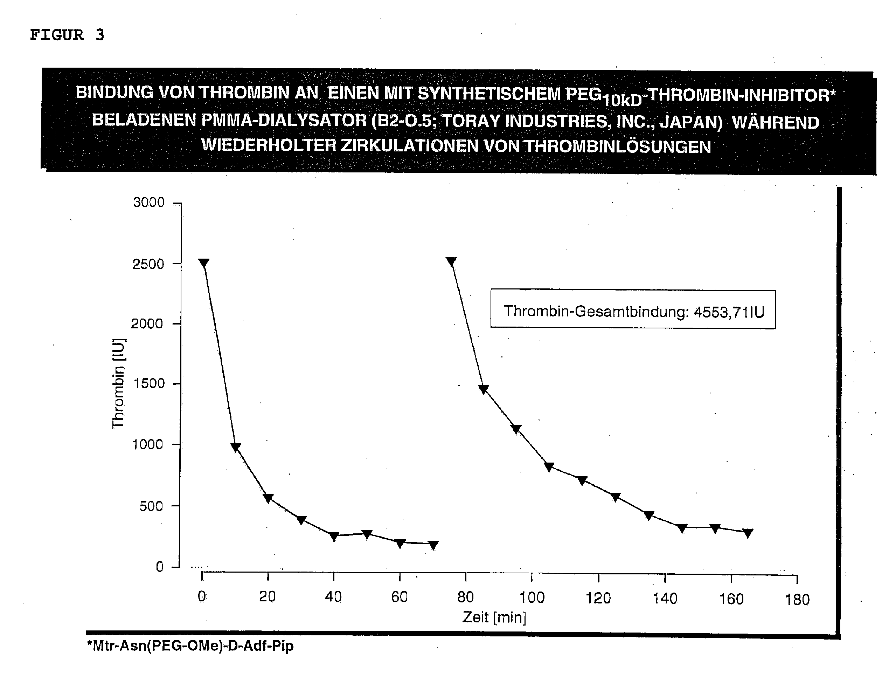

Eine Hämodialyse-Einheit vom Typ Fresenius A2008C wird zur Vorspülung der Blut- und Dialysatseite der Dialysatoren benutzt. Die PMMA-Dialysatoren werden nach der Vorschrift des Herstellers für die Dialyse vorbereitet. Am Ende der Vorspülung der PMMA-Dialysemembran wird der Zu- und Abfluß der Dialysatseite mit Schlauchklemmen total verschlossen, so daß kein Wechsel der Dialysatflüssigkeit während des Versuchs vorgenommen werden kann. Die an der Dialyse-Einheit vorhandene Schlauchpumpe wird mit einem Vorratsgefäß und mit der arteriovenösen Seite verbunden. In das silikonisierte Glas-Vorratsgefäß wird das abgenommene und PEG-Hirudin-antikoagulierte Rinderblut eingefüllt und unmittelbar danach mit der Rezirkulation der Blutseite begonnen. In kurzen Abständen werden Proben aus dem Blutreservoir entnommen und mit Hilfe der Ecarin-Gerinnungszeit (vgl. deutsche Patentanmeldung P 42 03 965.7) die Hirudinkonzentration im Rezirkulationssystem gemessen. Wie aus Figur 3 hervorgeht, kommt es mit dem Beginn der Zirkulation der PMMA-Membran zu einem raschen Abfall der Hirudinkonzentration im Blut, innerhalb der ersten 40 Minuten.

Die Figur 3 zeigt die Oberflächenbindung eines synthetischen kleinmolekularen Thrombininhibitors, der mit Polyethylenglykol (10 kD) nach Vorschrift von W. Stüber et al. (Peptide Research 8 (2) 1995, S. 78-85) als pegylierter Hemmstoff hergestellt wurde. Das wirksame Thrombininhibitorprinzip ist die Substanz Mtr-Asn-D-Adf-Pip, welche sich in vorangegangenen Untersuchungen (Dickneite G. et al. Thrombosis Research 77, S. 537-568) als hochspezifischer und stark wirksamer Hemmstoff des Thrombins erwiesen hat.

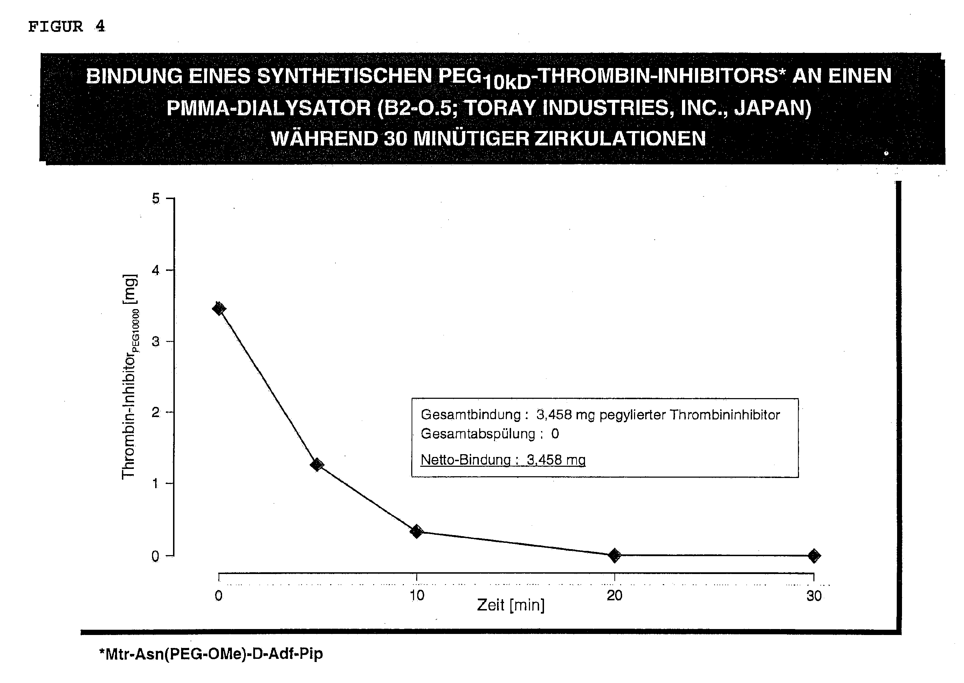

Die Figur 4 zeigt die Thrombinbindung an einen mit PEG-(10 kD)-Thrombininhibitor-beladenen PMMA-Dialysator. Aus dieser Abbildung ist ersichtlich, daß Thrombin während der Rezirkulation an den gekoppelten Thrombininhibitor anbindet und dementsprechend rasch aus der Rezirkulationslösung verschwindet.

Aus den hier dargestellten PEG-Hirudinkonzentrationen auf der Blut- und Dialysatseite ist erkennbar, daß auf beiden Seiten des Rezirkulationssystems kein meßbares PEG-Hirudin mehr vorhanden ist.

Zu Vergleichszwecken ist das Verhalten von rekombinantem Hirudin bei Verwendung von PMMA-Dialysatoren dargestellt. Es wurde mit 30-50 *µ*g/ml r-Hirudin antikoaguliertes Rinderblut verwendet. Aus der Abbildung ist ersichtlich, daß es ebenfalls zu einer Verminderung der Hirudinkonzentration auf der Blutseite kommt, die aber sehr rasch in einen steady-state hinübergeht. Bei der Untersuchung nach der zweistündigen Hämodialyse sind im Dialysatraum entsprechend hohe Konzentrationen von r-Hirudin wie auf der Blutseite vorhanden, d.h. es kommt zu einem typischen totalen r-Hirudin-Konzentrationsausgleich auf beiden Seiten. Eine adsorptive Bindung (Verlust von r-Hirudin) ist vernachlässigbar klein. Aus den hier vorgestellten Untersuchungen geht eindeutig hervor, daß PEG-Hirudin mit hoher Spezifität an PMMA-Kapillardialysatoren gebunden wird. Der direkte Vergleich mit dem ungebundenen r-Hirudin läßt zweifelsfrei den Schluß zu, daß der PEG-Anteil des Hirudins für die Kopplung an der PMMA-Oberfläche verantwortlich ist.

### Beispiel 4

### Herstellung und Verwendung eines Polyethylenglykol (20000) - gekoppelten monoklonalen Antikörpers gegen Tumornekrosefaktor (MAK 195)

100 mg MAK 195 werden in 50 ml 0,1-Mol Boratpuffer (pH 8) gelöst und mit 200 mg Methoxypolyethylenglykol (20000)-4-Nitrophenylcarbonat versetzt und für 3 h bei 25°C inkubiert. Die Kopplungsreaktion wird mit einem 500fachen molaren Überschuß an TRIS gestoppt, gegen 20 mMol TRIS-HCL dialysiert und auf einer HP-Q-Sepharose-Säule (Pharmacia) mit einem linearen NaCl-Gradienten (von 0-400 mMol NaCl) in 20 mMol TRIS-HCl (pH 8) aufgetragen. Das PEG-MAK 195-Konjugat eluiert etwa bei 200-250 mMol NaCl. Die Bindungsfähigkeit des monoklonalen Antikörpers wird an einem rekombinanten humanen α-TNF mit Hilfe eines α-TNF-ELISA's getestet. Ein Wirkungsverlust des monoklonalen Antikörpers kann nicht festgestellt werden. Der pegylierte monoklonale Antikörper gegen TNF wird auf ein experimentelles PMMA-Kapillardialysatormodul mit 50 cm² Oberfläche aufgebracht. Die Bindungsfähigkeit betrug für 50 cm² Oberfläche 4 mg, bezogen auf den Proteingehalt des PEG-MAK 195. In Humanplasma, welches mit TNF (100 ng/ml) gespikt wurde, ließ sich bereits nach 10 min in der Rezirkulationsflüssigkeit kein Tumornekrosefaktor mehr feststellen.

Eine weitere Versuchsserie wurde durchgeführt, indem 50 ml Humanblut PEG-MAK 195 in einer Konzentration von 1 mg pro ml zugegeben wurde. Diese PEG-MAK-Blutmenge wurde mittels Rezirkulation einem experimentellen PMMA-Kapillardialysator von 50 cm² Oberfläche zugeführt und mittels einer Schlauchpumpe rezyklisiert. Nach 30 min Perfusion war im vorgelegten Blut kein MAK-Antikörper mehr nachweisbar. Der Nachweis wurde mit Hilfe eines TNF-inhibitorischen Assays auf der Meßbasis eines TNF-ELISA's durchgeführt. Aus diesen beiden Versuchen kann zweifelsfrei nachgewiesen werden, daß sich pegylierter monoklonaler Antikörper, hier der TNF-Antikörper MAK 195 (Knoll), spezifisch auf der PMMA-Kapillardialysatormembran coatieren läßt. Er ist dort in der Lage, Human-TNFα quantitativ zu binden.

Im zweiten Versuch wurde nachgewiesen, daß ein im Blut vorhandener pegylierter monoklonaler Antikörper von der PMMA-Membran spezifisch aus der Zirkulation entfernt wird. Dieser Versuch beweist das "Fischen" eines für eine begrenzte Zeit im Blut zur Anwendung gekommenen pegylierten Wirkstoffs.

### Beispiel 5

### Verwendung eines PEG-gekoppelten Enzyms

Als Modellenzym wird die Urease (EC 3.5.1.5., zu beziehen von Sigma, Best.-Nr. U 1500) verwendet. Herstellung eines PEG-gekoppelten Ureasepräparats: 100 mg Urease (Typ III, spezifische Aktivität 20.000 bis 30.000 U/g) werden in 80 ml 0,1-Mol Boratpuffer (pH 8) gelöst und mit 250 mg Methoxypolyethylenglykol (25.000)-4-Nitrophenylcarbonat versetzt und für 24 h bei 5°C inkubiert. Die Reaktion wird mit einem mehrfach höheren molaren Überschuß an TRIS gestoppt, gegen TRIS-HCl (pH 8) dialysiert und in einer HP-Q-Sepharose-Säule mit einem linearen NaCl-Gradienten (pH 8) entwickelt. Das PEG-Urease-Konjugat eluiert bei 180 bis 200 mMol NaCl. Die Ausbeute des PEG-Urease-Konjugats liegt bei 30-40 %. Die Enzymaktivität.wird mit einem Harnstoff-Assay gemessen. Die Spaltungsaktivität wird mittels pH-Titration messend verfolgt und mit nicht konjugierter Urease verglichen. Die Pegylierung der Urease hat einen leichten aktivitätserhöhenden Einfluß auf die harnstoffspaltende Aktivität des Enzyms. Die mit 25 kD-Polyethylenglykol gekoppelte Urease wird in einer Konzentration von 2 mg (bezogen auf den Proteingehalt des Konjugats) auf einen miniaturisierten experimentellen Kapillardialysator mit 50 cm² Oberfläche aufgetragen. Bereits nach der ersten Zirkulation sind mehr als 90 % der Urease oberflächenfixiert. Bis zum fünften Rezirkulationszyklus ist die PEG-Urease fest auf der Oberfläche der Membran installiert. Eine Auswaschung mittels halbstündiger Spülung mit einer Albumin-Kochsalzlösung ergab keinen Aktivitätsverlust bzw. Auftreten von freier Ureaseaktivität in der Spülflüssigkeit. Auf das Urease-dotierte PMMA-Kapillardialysator-System wird eine Blutplasmalösung (50 ml enthaltend 30 mg Harnstoff) aufgetragen. Im Eluat der experimentellen PEG-Urease-PMMA-Membran werden nach 5, 10, 20 und 30 min die verbliebenen Harnstoffmengen mittels eines Enzymassays nachgewiesen. Es kann gezeigt werden, daß sehr rasch innerhalb der ersten 10 min die Harnstoffkonzentration im Blutplasma abfällt. Nach 30 min ist freier Harnstoff nicht mehr nachweisbar. Die Ammoniakprobe im Blut ist während der letzten 25 min des Versuchs positiv. Aus den Untersuchungen kann zweifelsfrei geschlossen werden, daß ein pegyliertes Enzym, auf die PMMA-Membran gebunden, eine lokale Enzymaktivität entfaltet und entsprechende spezifische Interaktionen mit seinem Substrat im zirkulierenden Blut erzeugen kann.

### Beispiel 6

### Herstellung und Verwendung von Polyalkylmethacrylat-Mikropartikeln

In weiteren Untersuchungen konnte nachgewiesen werden, daß auch Mikropartikel aus Polyalkylmethacrylat (PAMA) in der Lage sind, diese Polyethylenglykol-gekoppelten Substanzen an ihrer Oberfläche zu binden. Die PAMA-Mikropartikel werden in an sich bekannter Weise hergestellt und mit den jeweiligen Wirkstoffen beschichtet. Es wurden unterschiedliche Partikelgrößen untersucht. Mikropartikel aus Polyalkylmethacrylat zwischen 0,5 µm und 250 µm Durchmesser binden große Mengen von PEG-Wirkstoffen. Diese Partikel können in vielfältiger Weise verwendet werden:

### 1) Partikel in der Größe bis 20 µm Ø :

Diese Partikel können nach spezifischer PEG-Wirkstoff-Dotierung intravasal appliziert werden und verbleiben dann für längere Zeit in der Blutzirkulation. Es besteht die Möglichkeit, diese Partikel wieder aus dem Körper auszuschleusen, wenn sie magnetisch anregbare Verbindungen (z.B. Fe₂O₃) enthalten. Durch eine spezifische externe Behandlung des Blutes mit magnetischen Rückhaltesystemen sind diese Partikel am Therapieende wieder aus der Blutbahn zu entfernen. Hier könnten Prodrugs (Substanzen, die erst im Blut durch hier vorhandene Enzyme in die wirksame Form überführt werden) zum Beispiel an bestimmte Organe oder Krankheitsherde im Organismus herangebracht werden, um dort dann in hoher Konzentration zur Therapie zur Verfügung zu stehen.

### 1.1 Sauerstofftransportvehikel

Durch das Priming dieser PAMA-Partikel mit Polyethylenglykol-gekoppeltem Hämoglobin entstehen "künstliche" Erythrozyten mit hohem spezifischen Sauerstofftransportvermögen und genügend langen Verweilzeiten im Organismus.

### 1.2 Antivirale Partikel

Auf den PAMA-Partikeln werden spezifische polyklonale bzw. monoklonale Antikörper gegen Hüllproteine (hochspezifische Erkennungsstellen) der Viren geprimt. Die Partikel sind in der Lage, die Viren quantitativ zu "fangen" und aus dem Organismus zu entfernen.

### 1.3 Monoklonale Antikörper (MAB) tragende Partikel

MAB-Partikel gegen Tumor-Nekrose-Faktor-(TNF-α) können sowohl bei der Sepsis verwendet werden, aber auch bei schweren Infektionskrankheiten. Die MAB-Partikel "finden" den von den infizierten Zellen produzierten TNF-α, der bei bestimmten Infektionen auch nach Endotoxin-Kontakt in hohen Konzentrationen freigesetzt wird.

### 1.4 Partikel-beschichtete Festkörper und Gewebe

### 1.4.1 Gelenk- und Knochenersatzprothesenmaterial

Monodisperse Partikel in der bevorzugten Größe 1 µm, 5 µm oder 10 µm werden mit geeignetem Klebefilm ("PAMA-Kleber") mit dem Ersatzmaterial fest verbunden. Nach Aushärtung der Klebeverbindung werden die PAMA-Partikel-beschichteten Materialien mit Polyalkylglykol-gekoppelten Wachstumsfaktoren für Knochen-, Bindegewebs- oder Muskelzellen (z.B. rBMP-2 oder rBMP-4, bFGF u.a.) geprimt und danach in den Körper eingebracht. Damit wird es erstmals möglich, feste Halteverbindungen zwischen Prothesenmaterial und Muskel zu erreichen (wiederherstellende Traumatologie!).

### 1.4.2 Gefäßprothesen

Goretex- oder Dacron-Gefäßprothesen werden mit einer PAMA-Partikel (0,5 µm Ø)-Schicht beklebt und vor Anwendung mit pegyliertem endothelialen Wachstumsfaktor geprimt. Durch die zusätzliche Anwendung von basischem Fibroblasten-Wachstumsfaktor, der zuvor PEG-gekoppelt wurde, ist eine bessere Vitalisierung durch bindegewebliche und mikrokapillare Wachstumsinduktion möglich.

### 1.4.3 Künstliche Organe

Biokompatible Gewebeschichten aus Kunststoffmaterialien, die mit monodispersen PAMA-Pattikeln beklebt wurden, oder Copolymere mit PAMA als Grundmaterial werden mit organspezifischen pegylierten zellulären Wachstumsfaktoren geprimt. Dadurch ist eine schnelle und gesteuerte Beschichtung der Organersatzmaterialien möglich.
Einsatzgebiete: Leberersatz, Knochenmarkersatz, Lungengewebeersatz u.a.

### 1.4.4 Zell-Chip-Kontakte

Biosensoren, wie z.B. Silicium-Chips und andere elektronische Schaltkreismaterialien können lokal mit monodispersen PAMA-Partikeln (bevorzugt 0,5 bis 5 µm Ø) beschichtet werden. Durch das Priming mit pegylierten Zellwachstumsfaktoren ist ein Aufwachsen von speziellen Zellendigungen möglich. Durch diese Technik kann ein Informationsaustausch zwischen Gewebe, bevorzugt Nervengewebe und intelligenten Schaltkreisen erreicht werden.

### 2) Partikel im Bereich 30 bis 80 µm Ø:

Diese Partikel können mit einer Vielzahl von Wirkstoffen, Enzymen, Antikörpern, Antithrombotika (direkte und indirekte), wie Hirudin, synthetischen kleinmolekularen Thrombin-inhibitoren, Protein-C-aktivierenden Schlangengiften aber auch Thrombolytika, wie Fibrinolase, tPA, Streptokinase-Aktivator-Komplex u.a., geprimt werden. Diese Partikel werden in miniaturisierten "Schlauchsäulen" direkt in einen venovenösen (passiv) bzw. arteriovenösen (aktiv) Shunt eingesetzt.

Die Größe der Partikel ist so bemessen, daß die korpuskulären Bestandteile des Blutes ungehindert passieren können. Die innere Oberfläche dieser Module ist so groß, daß selbst intravasale "temporäre" Systeme möglich sind.

Durch das "Mehrfach"-Priming von PAMA-Oberflächen ist es möglich, spezifische enzymatische Reaktionen im Blut zu induzieren. Hierbei könnten auch Leukozyten, Monozyten, polymorphkernige weiße Blutzellen an der Oberfläche der ETS spezifisch verändert werden (z.B. Abspaltung von entzündungsspezifischen Determinanten).

### 3) Partikel im Bereich 80 bis 150 µm ∅:

Diese Partikelgrößen sind besonders für den Magen-Darm-Kanal bzw. die orale Anwendung geeignet. Durch die große innere Oberfläche und die problemlose Anwendung zur gezielten Modifikation von Verdauungsvorgängen kann das Cholesterin z.B. bereits vor der Resorption metabolisch-chemisch so verändert werden, daß es nicht mehr bzw. nur noch in sehr geringer Menge resorbiert werden kann. Dadurch wird der entero-hepatische Kreislauf des Cholesterins unterbrochen.

Die Verwendung von oberflächengebundenen Alkoholdehydrogenasen ist eine weitere wichtige Indikation, um den Blutalkoholspiegel zu senken.

### 4) Partikel im Bereich 120 bis 280 µm Ø:

Diese Partikelgröße ist bevorzugt für die orale Applikation von diätetischen Wirkstoffen geeignet. Durch die große innere Oberfläche der Partikel ist ein intensiver Besatz mit verdauungsmodifizierenden Enzymen möglich (Fett- und Cholesterin-abbauende Enzyme oder -bindende Strukturen). Die Bindung von pegylierter Alkoholdehydrogenase kann im Magen-Darm-Kanal die Alkoholingestion vermindern.

## Patentansprüche

1. Verwendung einer Oberfläche aus einem Kunststoff auf der Basis von Monomeren, die mindestens ein von einer Carbonsäure abgeleitetes Strukturelement (A) enthalten, wobei der Kunststoff ein Poly(meth)acrylat, ein Polyvinylester oder ein Mischpolymer oder Gemisch davon mit anderen polymersierbaren Substanzen ist zur Entfernung eines Linkers mit einer daran gekoppelten physiologisch und/oder pharmakologisch aktiven Substanz aus einer Flüssigkeit, wobei der Linker ausgewählt ist aus (Poly)alkylenglykolen, (Poly)alkyleniminen, (Poly)alkylenaminen, (Poly)alkylensulfiden und Polyoxazolinen und mindestens ein Strukturelement (B), das zur Bildung von Wasserstoffbrücken fähig ist, aufweist, wobei über Wasserstoffbrückenbindungen eine stabile Wechselwirkung zwischen dem Strukturelement (A) der Oberfläche und dem Strukturelement (B) des Linkers entsteht, die durch pH-Werte im Bereich von 2 bis 13 oder Temperaturen bis 60°C nicht aufgehoben werden kann.

2. Verwendung nach Anspruch 1, wobei die aktive Oberfläche eine Membran, ein poröses oder solides Mikropartikel, ein magnetisches Mikropartikel, eine Filtermatte, ein faserförmiges Material, ein versponnenes Material oder eine Kombination hiervon, oder eine Beschichtung auf einer natürlichen oder synthetischen Substanz ist.

3. Verwendung nach Anspruch 1 oder 2, wobei die aktive Substanz ein Protein, eine Nukleinsäure, ein zellulärer Signalstoff, ein Partner eines biologischen oder physiologischen Affinitätspaares, ein synthetischer Ni-NTA-Komplex, ein synthetisch hergestelltes Pharmakon bzw. ein synthetisch hergestellter Wirkstoff oder ein Marker für eine biologische oder synthetische Substanz ist.

4. Verwendung nach Anspruch 3, wobei das Protein ausgewählt ist aus Enzymen, Antigenen, Antikörpern, Tumormarkern, Oberflächenantigenen, Liganden, Rezeptoren, oberflächenaktiven Zellfragmenten von Bakterien oder Viren oder Immunbotenstoffen.

5. Verwendung nach Anspruch 3, wobei der Marker eine fluoreszierende oder chemilumineszierende Substanz, ein EST (Expressionssequenz-Code) oder ein Enzym ist.

6. Verwendung nach Anspruch 1 oder 2, wobei die aktive Substanz ein Gerinnungshemmstoff, ein stoffwechselaktives Enzym, ein Antibiotikum oder ein synthetischer Arzneistoff ist.

7. Verwendung nach einem der Ansprüche 1 bis 6, wobei der Kunststoff Poly(meth)acrylat oder ein Poly(meth)acrylat-Mischpolymeres ist, der Linker Polyethylenglycol ist und die gekoppelte Substanz ein Antikoagulans, ausgewählt aus Heparin, Hirudin, direkt wirkenden Antithrombinen oder Prothrombin, ist.

8. Verwendung nach einem der Ansprüche 1 bis 6, wobei der Kunststoff Poly(meth)acrylat oder ein Poly(meth)acrylat-Mischpolymeres ist, der Linker Polyethylenglykol ist und die gekoppelte Substanz ein Enzym oder ein Arzneimittel ist.

9. Verwendung nach einem der Ansprüche 1 bis 8, wobei die Flüssigkeit eine biologische Flüssigkeit, ausgewählt aus Vollblut, Plasma, Serum, Urin, Lymphe, Liquor, Organbioptat, Darminhalt oder Sperma, ist.

10. Verwendung nach einem der Ansprüche 1 bis 8, wobei die Flüssigkeit ein Lebensmittel oder Lebensmittelbestandteil ist.

11. Verwendung nach einem der Ansprüche 1 bis 8 als extrakorporales therapeutisches oder diagnostisches System.

12. Verwendung nach Anspruch 11 zur Diagnose und Therapie von Stoffwechselerkrankungen, Gerinnungsstörungen, viralen, bakteriellen, mykotischen oder parasitären Infektionen oder malignen Erkrankungen.

13. Verwendung nach Anspruch 10 zur Herstellung von diätischen Lebensmitteln oder Design-Food.

14. Verwendung nach einem der Ansprüche 1 bis 8 zur Herstellung nach Nachweissystemen.

15. Verwendung nach Anspruch 14 zur Herstellung von Affinitätssäulen, Mikrotiterplatten, ELISA-Testplatten, Mikrosticks, Diagnosesticks, Hohlfasern oder Flächengebilden.
